# FASCICULE DE BREVET EUROPEEN

(11) **EP 2 697 652 B1**
(45) Date de publication et mention de la délivrance du brevet: **14.09.2016**
(21) Numéro de dépôt: 12722412.9
(22) Date de dépôt: 12.04.2012
(51) Int. Cl.: G01N 33/68

(54) **DIAGNOSTIC ET TRAITEMENT DE L'ANGIOEDÈME**
DIAGNOSE UND BEHANDLUNG VON ANGIOÖDEMEN
DIAGNOSIS AND TREATMENT OF ANGIOEDEMA

(30) Priorité: 14.04.2011 FR 1153239
(43) Date de publication de la demande: 19.02.2014
(73) Titulaire: Universite Joseph Fourier (Grenoble 1), 38041 Grenoble (FR); Centre Hospitalier Universitaire de Grenoble, 38043 Grenoble Cedex 9 (FR)
(72) Inventeur: DROUET, Christian, F-38240 Meylan (FR); LUYASU, Samuel, B-6760 Saint-Remy (BE); GHANNAM, Arije, F-38000 Grenoble (FR)
(74) Mandataire: Agasse, Stéphane
(86) Numéro de dépôt international: PCT/FR2012/050804
(87) Numéro de publication internationale: WO 2012/140372

(56) Documents cités:
- WO-A1-97/09347
- US-A- 5 700 779
- SABINA ANTONELA ANTONIU: "Therapeutic Approaches in Hereditary Angioedema", CLINICAL REVIEWS IN ALLERGY & IMMUNOLOGY, HUMANA PRESS INC, NEW YORK, vol. 41, no. 1, 29 janvier 2011 (2011-01-29), pages 114-122, XP019933932, ISSN: 1559-0267, DOI: 10.1007/S12016-011-8254-2
- LONGHURST HILARY J: "Management of acute attacks of hereditary angioedema: potential role of icatibant.", VASCULAR HEALTH AND RISK MANAGEMENT 2010 LNKD- PUBMED:20859548, vol. 6, 2010, pages 795-802, XP002661276, ISSN: 1178-2048
- MOLINARO GIUSEPPE ET AL: "Biochemical basis of angioedema associated with recombinant tissue plasminogen activator treatment: An in vitro experimental approach", STROKE, vol. 33, no. 6, juin 2002 (2002-06), pages 1712-1716, XP002661277, ISSN: 0039-2499
- CYR MELANIE ET AL: "Bradykinin metabolism and hypotensive transfusion reactions", TRANSFUSION (BETHESDA), vol. 41, no. 1, janvier 2001 (2001-01), pages 136-150, XP002661278, ISSN: 0041-1132
- JOSEPH K ET AL: "Studies of the mechanisms of bradykinin generation in hereditary angioedema plasma", ANNALS OF ALLERGY, ASTHMA & IMMUNOLOGY, ARLINGTON HEIGHTS, IL, US, vol. 101, no. 3, 1 septembre 2008 (2008-09-01), pages 279-286, XP026960155, ISSN: 1081-1206 [extrait le 2008-09-01]
- RAYMOND PHILIPPE ET AL: "Quantification of des-Arg-9-bradykinin using a chemiluminescence enzyme immunoassay: Application to its kinetic profile during plasma activation", JOURNAL OF IMMUNOLOGICAL METHODS, ELSEVIER SCIENCE PUBLISHERS B.V.,AMSTERDAM, NL, vol. 180, no. 2, 1 janvier 1995 (1995-01-01), pages 247-257, XP002494580, ISSN: 0022-1759, DOI: 10.1016/0022-1759(94)00320-V
- MOLINARO GIUSEPPE ET AL: "Angiotensin-converting enzyme inhibitor-associated angioedema is characterized by a slower degradation of des-arginine(9)-bradykinin.", THE JOURNAL OF PHARMACOLOGY AND EXPERIMENTAL THERAPEUTICS OCT 2002 LNKD- PUBMED:12235256, vol. 303, no. 1, octobre 2002 (2002-10), pages 232-237, XP002661279, ISSN: 0022-3565
- SCHAPIRA M ET AL: "HIGH MOLECULAR WEIGHT KININOGEN OR ITS LIGHT CHAIN PROTECTS HUMAN PLASMA KALLIKREIN FROM INACTIVATION BY PLASMA PROTEASE INHIBITORS", BIOCHEMISTRY, vol. 21, no. 3, 1982, pages 567-572, XP002661280, ISSN: 0006-2960

## Description

La présente demande de brevet concerne une méthode de diagnostic de l'angioedème à gain-de-fonction de la kininoformation et de la production de bradykinine (BK) au cours de la crise pour la détermination de la quantité d'antagoniste de la BK nécessaire pour stopper les effets d'un angioedème, à partir d'un échantillon de plasma d'un patient. L'invention concerne également un kit pour cette détermination.

L'angioedème (AO) est une situation pathologique s'exprimant par une fuite capillaire, avec gonflement des tissus sous-cutanés ou sous-muqueux. Cette fuite capillaire est engendrée par la production de BK, par coupure de son précurseur, le kininogène de haut poids moléculaire (HK) par la kallicréine plasmatique. La BK exerce ses effets par la stimulation du récepteur B2 des kinines exprimé sur les cellules endothéliales. La perméabilité vasculaire entraîne chez le patient en situation de crise des crampes abdominales accompagnées parfois de vomissements et de diarrhées. La crise provoque également une gêne respiratoire qui peut aller jusqu'à l'asphyxie en cas de localisation laryngée. Dans certains cas sévères, l'angioedème peut conduire à la mort du patient.

Les sujets en situation de crise sont admis en service d'urgence. Le diagnostic est difficile par la symptomatologie commune avec l'allergie, l'oedème etle choc anaphylactique. L'un des traitements d'urgence de ces patients est l'icatibant, un antagonistedu récepteur B2 de la BK par voie sous-cutanée dans la région abdominale. L'injection est destinée à stopper les effets de la crise. L'icatibant est conditionné sous forme d'une solution injectable de 30 mg (10mg/ml). Ce conditionnement représente une quantité excédentaire. Ceci est principalement dû au fait qu'aucune méthode visant à déterminer la quantité d'icatibant nécessaire à stopper les effets d'une crise n'est aujourd'hui disponible.

Malgré ce défaut de connaissance, injecter une quantité excédentaire d'icatibant assure aujourd'hui l'efficacité dans le traitement en urgence d'un patient en situation de crise aiguë. Néanmoins, cela présente également un certain nombre d'inconvénients. Un de ceux-ci est la survenue chez le patient d'effets secondaires indésirables, et notamment de réactions aux sites d'injections (rougeurs, sensations de brulures, douleurs) chez pratiquement tous les patients. Un autre de ces inconvénients est la récurrence de la crise chez une partie des patients. L'icatibant présente en outre un prix de vente très élevé et il est certain qu'une posologie plus adaptée diminuerait le coût du traitement et donc les dépenses de santé occasionnées.

Il serait donc particulièrement avantageux de disposer d'une méthode qui permette, au cas par cas, à partir d'un échantillon de sang du patient et en fonction de la sévérité de la crise, de déterminer la quantité d'icatibant nécessaire pour stopper les effets de cette crise. La présente invention s'inscrit dans ce contexte.

L'absence de diagnostic fondé sur le métabolisme de la kininoformation amène également la présente invention à un développement dans ce sens.

La présente invention permet donc le diagnostic, le suivi, le pronostic et le traitement de l'angioedème à gain-de-fonction.

### Description de l'invention

La présente invention concerne une méthode de détermination de la quantité d'antagoniste de la BK nécessaire pour stopper les effets d'un angioedème chez un patient comprenant :
a) l'activation de la libération de BK par mise en contact d'un échantillon du plasma du patient avec une quantité déterminée d'un agent électronégatif,
b) le dosage de la quantité de BK libérée selon l'étape a), qui correspond à la réserve de BK dans l'échantillon de plasma du patient,
c) la comparaison de la valeur obtenue à l'étape b) avec une valeur contrôle qui correspondà la réserve de BK disponible dans un échantillon de plasma d'un témoin sain de même catégorie, en vue de la détermination de la quantité de BK produite dans l'échantillon de plasma du patient,
d) la détermination de la quantité totale de BK produite chez le patient, et
e) la détermination de la quantité nécessaire dudit antagoniste, à partir de la détermination réalisée à l'étape e), cette quantité étant équimolaire avec la quantité de BK produite.

Selon un aspect de l'invention, le dosage de l'étape b) est un dosage immunoenzymatique compétitif.

Selon un autre aspect de l'invention, on utilise, pour le dosage de l'étape b), des anticorps anti-BK, des traceurs DIG couplés à la BK, des anticorps anti-DIG couplés à une enzyme et des substrats de l'enzyme.

Selon un autre aspect de l'invention encore, l'étape b) comprend elle-même les étapes suivantes :
- on immobilise des anticorps anti-BK dilués dans du tampon de fixation sur un support approprié comportant plusieurs cavités ;
- à intervalle de temps régulier, on prélève une partie aliquote de l'échantillon activé et on incube une cavité du support avec ladite partie aliquote de l'échantillon et une quantité déterminée d'un traceur DIG couplé à la BK pendant une période de temps déterminée;
- après lavage, on incube le support avec des anticorps anti-DIG couplés à une enzyme ;
- après lavage, on incube le support avec une quantité déterminée du substrat de l'enzyme ;
- on mesure l'activité enzymatique par mesure colorimétrique directe.
L'enzyme couplée aux anticorps anti-DIG peut par exemple être la phosphatase alkaline ou la peroxidase.

Selon un aspect de l'invention, l'antagoniste de la BK est l'icatibant (HOE 140).

L'invention concerne aussi une méthode de diagnostic d'un angioedème avec forte kininoformation chez un patient comprenant :
a) l'activation de la libération de BK par mise en contact d'un échantillon du plasma du patient avec une quantité déterminée d'un agent électronégatif,
b) le dosage de la quantité de BK libérée selon l'étape a), qui correspond à la réserve de BK dans l'échantillon de plasma du patient,
c) la comparaison de la valeur obtenue à l'étape b) avec une valeur contrôle qui correspondà la réserve de BK disponible dans un échantillon de plasma d'un témoin sain de même catégorie, en vue de la détermination de la quantité de BK produite dans l'échantillon de plasma du patient, et
d) la détermination de la quantité totale de BK produite chez le patient.
e) une forte diminution de la quantité de BK libérée par activation dans l'échantillon de plasma du patient comparativement à la quantité déterminée dans les mêmes conditions dans l'échantillon de plasma du témoin atteste d'un angioedème avec forte kininoformation, dont l'angiodème à gain de fonction.

### Description des figures

Figure 1 : Formation de la bradykinine endothéliale (kininoformation).

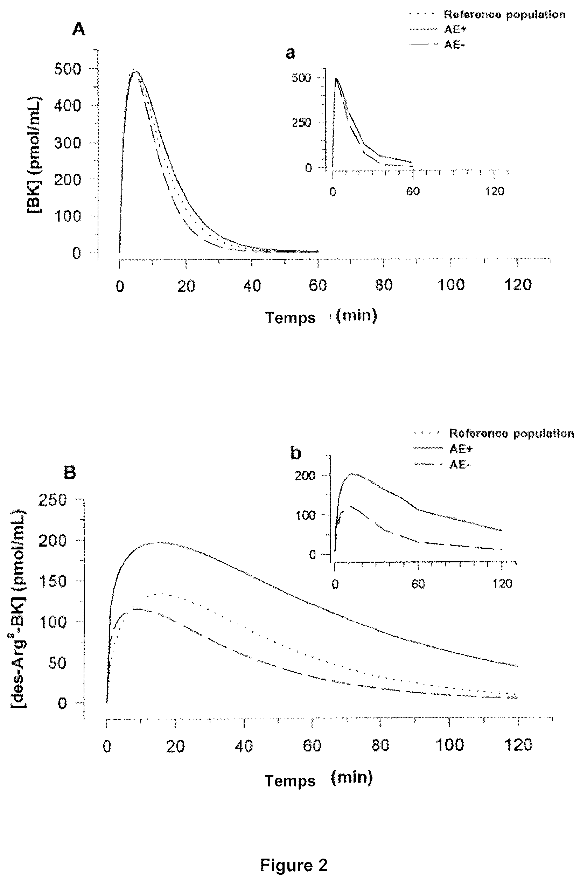

Figure 2 : Capacité de formation des kinines chez les patients souffrant d'angioedème. Le plasma est soumis à l'activation *in vitro* par une surface chargée - (billes de verre), puis les concentrations de BK (A) et desArg⁹-BK (B) sont suivies dans le temps (sur 60 minutes). AE+, population souffrant d'angioedème suite aux thérapeutiques par inhibiteurs d'enzyme de conversion de l'angiotensine-I ; AE-, population normale sans signe pathologique et soumise à la même thérapeutique ; référence population, population normale de référence.
Figure 3 : Evénements biologiques (kininogénase spontanée et potentiel d'activabilité) avant la crise, pendant la crise et à distance de 21 jours de la crise chez un patient
Figure 4 : Evolution de la capacité du kininogène HK à produire BK et desArg⁹-BK dans deux situations : avant et après la crise d'angioedème à gain de fonction (figures 4A, 4B)

### Méthode de détermination de la quantité d'antagoniste de la bradykinine nécessaire pour stopper les effets d'un angioedème

La présente invention concerne donc une méthode de détermination de la quantité d'antagoniste de la BK nécessaire pour stopper les effets d'un angioedème chez un patient. Cette méthode comprend cinq étapes essentielles. La méthode selon l'invention est réalisée à partir d'un échantillon de plasma citraté du patient. Ainsi, à titre d'exemple, le volume de l'échantillon de plasma du patient est de 500µL.

Selon une première étape, on active la libération de BK par mise en contact de l'échantillon de plasma du patient avec une quantité déterminée d'agents électronégatifs. Cela a pour effet l'activation des protéases de la phase contact, avec coupure du kininogèneHK présent dans le plasma. Cette coupure s'exerce vis-à-vis de deux liaisons peptidiques (Séquence ID P01042 - avant le résidu Arginine en position 380 et après le résidu Arginine en position 388) pour donner la BK.

Par « patient », on entend un individu humain qui se trouve en situation de crise d'angioedème ou dont on suspecte qu'il est en préparation de crise d'angioedème.

Par « agent électronégatif », on entend un agent susceptible d'activer la phase contact du plasma, système de production de la BK. Il peut s'agir d'un produit solide ou d'un produit soluble par exemple. On peut citer l'utilisation de sulfate de dextran, de billes de verre ou de silicate d'aluminium (kaolin, Al₂Si₂O₅(OH)₄).

Par « quantité déterminée d'agents électronégatifs », on entend la quantité nécessaire d'agents électronégatifs pour activer la phase contact dans le volume de plasma considéré dans l'expérience; cette quantité est excédentaire par rapport à la quantité des pro-enzymes de la phase contact et est déterminée par observation de la courbe dose-réponse (Kluft, 1978 ; Müller, 2009). Si, par exemple, le choix de l'agent électronégatif est porté sur le sulfate de dextran, la concentration requise de l'agent électronégatif est de 25µg/ml.

Selon une seconde étape de la méthode de l'invention, on procède au dosage de la quantité de BK libérée selon l'étape a), qui correspond à la réserve de BK dans l'échantillon de plasma du patient. Par « réserve de BK », on entend la quantité de BK disponible dans l'échantillon de plasma, c'est-à-dire libérable par activation de la phase contact. Une comparaison de cette valeur, à une valeur contrôle (étape c) de la méthode selon la présente invention) permet d'évaluer la quantité de BK que le patient a produit au cours de l'évènement pathologique, et donc de déterminer la quantité d'antagoniste qui doit être administrée au patient pour stopper les effets de la crise.

Joseph *et al.* (2008) ont décrit la comparaison de la quantité de BK dans le plasma d'individus sains avec celle d'individus atteints d'angiodème héréditaire à l'aide d'un EIA.

Cette quantité de BK peut notamment être exprimée en unité de poids par unité de volume de plasma (par exemple en ng/ml) ou en nombre de moles par unité de volume de plasma (par exemple en nmol/l).

Selon un aspect de l'invention, le dosage de la BK est réalisé au moyen d'un dosage immunoenzymatique compétitif.

Par « dosage immunoenzymatique compétitif », on entend un dosage quantitatif incluant la mesure d'un standard calibré et mettant en oeuvre une réaction de compétition vis-à-vis du standard en utilisant un traceur.

Par « traceur », on entend entité mimant le standard antigène et auquel on adjoint une molécule permettant son traçage. Les traceurs sont par exemple produits par couplage de l'antigène à la DIG (digoxigénine) en partie amino-terminale.

Selon un aspect de l'invention, le dosage de la quantité de BK comprend une ou plusieurs étapes. Lorsque ce dosage est réalisé en plusieurs étapes, l'échantillon de plasma et les traceurs sont mis en contact avec un anticorps (anticorps de capture) reconnaissant spécifiquement la BK. Ensuite, l'antigène capturé (incluant la BK à doser et le traceur) est mis en contact avec un anticorps (anticorps de révélation). Lorsque le dosage est réalisé en une étape, l'antigène testé (incluant la BK à doser et le traceur) est mis en contact avec l'anticorps de révélation simultanément dans le puits contenant l'anticorps de capture.

Raymond *et al.* (1995) ont décrit une méthode de détermination de la quantité de BK dans un échantillon de plasma à l'aide d'un EIA.

Molinaro *et al.* (2002) ont eux, décrit la quantification de la *des*Arg⁹-BK à l'aide du test décrit par Raymond et al. (1995).

Des anticorps spécifiques peuvent être obtenus selon des techniques décrites par exemple dans Czernik AJ, et al (1991) Production of phosphorylation state-specificantibodies. MethodsEnzymol 201: 264-283. On peut pour ce faire, immuniser des animaux, par exemple des lapins, avec la partie carboxy-terminale de la BK qui doit être suffisante pour constituer unépitope. On choisit de préférence des anticorps qui présentent une forte affinité et une bonne spécificité pour l'antigène.

Si nécessaire, le dosage peut être réalisé en mettant en oeuvre un second anticorps reconnaissant le premier anticorps, pour améliorer la spécificité.

Par « anticorps », on entend un anticorps comprenant l'ensemble des immunoglobulines, ou un fragment de ces immunoglobulines contenant ou constitué par le site de reconnaissance pour l'antigène cible, par exemple, un fragment constitué par une chaîne lourde ou par l'association des chaînes lourdes et légères, ou un fragment de celles-ci par exemple un fragment (Fab)'2 ou un fragment Fab.

La révélation de la formation du complexe immunologique est effectuée avec un substrat du marqueur de l'anticorps par exemple un substrat coloré ou un substrat fluorescent et comprend une étape de mesure de la densité optique. Un marqueur tel que la peroxydase ou la phosphatase alcaline peut être utilisé.

La mesure de la densité optique est par exemple réalisée à 450nm notamment lorsque le marqueur de l'anticorps est une peroxydase.

Des anticorps spécifiques de la BK ou de la desArg⁹-BK sont disponibles dans le commerce ou peuvent être préparés par les méthodes citées dans la présente demande.

Par exemple, les anticorps suivants sont disponibles auprès des sociétés suivantes :
(1) antibodies-online GmbH, SchlossRaheStr. 15, D-52072 Aachen, Allemagne, Fax: +33 8111 49 891, Tel: +33 8111 49 890,
(2) Abcam, 24 rue Louis Blanc, 75010 Paris
(3) AbDSerotecMorphoSys UK Ltd, Endeavour House, Langford Business Park, Langford Lane, Kidlington, Oxford, GB-OX5 1 GE, UK
(4) Bachemdistribution Services GmbH, HegenheimerStrasse 5, D- 79576 Weil am RheinAllemagne

Pour la réalisation du dosage selon l'invention, l'échantillon de sang prélevé chez un patient est traité de façon à prévenir la possibilité qu'il coagule. En particulier, on y ajoute un agent anticoagulant habituel pour la réalisation des dosages *in vitro* effectués sur le sang, par exemple de l'héparine ou du citrate de sodium, tel que le citratetrisodique par exemple aux concentrations de 0,109M ou 0,129M suivant les proportions de 9 volumes de sang pour 1 volume d'anticoagulant.

On peut, par exemple, utiliser pour le dosage de la BK des anticorps anti-BK, des traceurs DIG couplés à la BK, des anticorps anti-DIG couplés à une enzyme et des substrats de l'enzyme.

Selon un mode réalisation de l'invention, le dosage de la BK comprend les étapes suivantes :
- on immobilise des anticorps anti-BK dilués dans du tampon de fixation sur un support approprié comportant plusieurs cavités ; par support approprié, on entend notamment, une plaque solide, par exemple une plaque 96-puits ;
- à intervalle de temps régulier, on prélève une partie aliquote de l'échantillon activé et on incube une cavité du support avec ladite partie aliquote de l'échantillon et une quantité déterminée d'un traceur DIG couplé à la BK pendant une période de temps déterminée ; on utilise par exemple les quantités suivantes de traceurs : 10-20 pg de BK-DIG (0.1-0.6pg de *des*Arg⁹-BK-DIG dans le cas du dosage de *des*Arg⁹-BK);
- après lavage, on incube le support avec des anticorps anti-DIG couplés à une enzyme ; par exemple, l'enzyme couplée aux anticorps anti-DIG est la phosphatase alkaline ou la peroxydase HRP;
- après lavage, on incube le support avec une quantité déterminée du substrat de l'enzyme ;
- on mesure l'activité enzymatique par mesure colorimétrique directe.

Par exemple, le dosage est réalisé comme suit : d'abord les immunoglobulines G anti-BK dont déposées dans les puits d'une plaque 96-puits. Ensuite, l'échantillon à doser ou le standard est mis en compétition avec une quantité connue de BK couplé au traceur (DIG). Par la suite, des anticorps anti-DIG couplé à l'HRP sont déposés dans chacun des puits. Finalement, la présence de complexe immun est révélée par la mesure de l'activité de la peroxydase. Plus précisément, une solution contenant de l'o-phénylènediamine est incubé dans chaque puits. L'absorption du substrat oxydé est lue à 450nm.

Selon une troisième étape de la méthode de l'invention, on compare, plus précisément on soustrait, la quantité de BK libérée (valeur obtenue à l'étape b) de la méthode selon l'invention) avec une valeur contrôle qui correspond à la réserve de BK disponible dans un échantillon de plasma d'un témoin sain de même catégorie, le volume de l'échantillon de plasma du témoin étant identique à celui de l'échantillon du patient. Ainsi, par exemple, si le dosage de l'étape b) a été réalisé à partir d'un volume de plasma de 500 µL, la comparaison doit être faite avec une valeur contrôle obtenue à partir d'un volume identique, c'est-à-dire 500 µL.

Par « témoin sain de même catégorie », on entend un témoin qui ne se trouve pas en situation de crise d'angioedème. Ce témoin doit être de même catégorie, c'est-à-dire qu'il doit être de même sexe et présenter un poids (en kg) et une taille (en cm) approximativement identiques, des variations de 10% étant toutefois acceptables. Ce témoin peut être un individu différent ou il peut s'agir de la même personne, le dosage ayant été réalisé à un moment où le patient n'était pas en situation ou en préparation de crise.

Ainsi, selon cette étape, on compare la réserve de BK dans un volume d'échantillon de plasma d'un patient avec la réserve de BK dans un volume identique de plasma d'un témoin sain de même catégorie. En d'autres termes, on compare la réserve de BK du patient à une valeur contrôle. Plus précisément, cela permet d'évaluer la quantité de BK qui est mobilisée par le processus de crise, évènement pathologique, d'angioedème chez le patient par rapport à un témoin sain.

Ainsi, on détermine la quantité de BK produite dans l'échantillon de plasma du patient, à partir de la valeur obtenue à l'étape de dosage dans l'échantillon du patient (réserve du patient) et de la valeur contrôle dans un volume plasmatique identique d'un témoin sain (réserve du témoin sain). En d'autres termes, la quantité réserve BK du patient est soustraite à la quantité réserve BK du témoin, de manière à évaluer la quantité de BK que le patient a produit au cours de l'événement pathologique. Cette quantité de BK peut notamment être exprimée en unité de poids par unité de volume de plasma (par exemple en ng/ml) ou en nombre de moles par unité de volume de plasma (par exemple en nmol/l).

Selon une quatrième étape, on détermine la quantité totale de BK produite chez le patient. Il s'agit d'une étape qui consiste à évaluer la quantité de BK produite dans le volume plasmatique total du patient (priseen compte le poids du sujet, de son volume sanguin et son volume plasmatique) à partir de la détermination réalisée à l'étape précédente, c'est-à-dire à partir de la quantité de BK dans un volume connu de plasma.

Selon cette étape, il convient de rapporter la valeur obtenue à la troisième étape, qui correspond à une quantité de BK dans un volume d'échantillon de plasma déterminé, par exemple 500 µL, au volume plasmatique total du patient, par exemple 2,25L, par une règle de trois.

Cette quantité peut notamment être exprimée en poids (par exemple en mg) ou en nombre de moles (par exemple en nmol). Selon une cinquième étape, on détermine la quantité nécessaire de l'antagoniste, à partir de la détermination réalisée à la quatrième étape, cette quantité étant équimolaire avec la quantité de BK consommée.

En d'autres termes, le nombre de moles d'antagoniste nécessaire pour stopper les effets de l'angioedème chez le patient est égal au nombre de moles de BK déterminé selon la quatrième étape de la méthode selon l'invention. Ce nombre de moles est ensuite converti en poids d'antagoniste qui doit être administré au patient, en considération du poids moléculaire de l'antagoniste. Par exemple, le poids moléculaire de l'antagoniste icatibant s'élève à 1305 g/mol.

### Méthode de détermination de la quantité d'antagoniste de nécessaire pour stopper les effets d'un angioedème

La présente invention concerne également une méthode de détermination de la quantité d'antagoniste de la desArg⁹-BK nécessaire pour stopper les effets d'un angioedème chez un patient comprenant les étapes suivantes :
a) on active la libération de *des*Arg⁹-BKpar mise en contact d'un échantillon du plasma du patient avec une quantité déterminée d'un agent électronégatif,
b) on dose la quantité de *des*Arg⁹-BKlibérée selon l'étape a), qui correspond à la réserve de *des*Arg⁹-BK dans l'échantillon de plasma du patient,
c) on compare la valeur obtenue à l'étape b) avec une valeur contrôle qui correspondà la réserve de *des*Arg⁹-BKdisponible dans un échantillon de plasma d'un témoin sain de même catégorie, de manière à déterminer la quantité de *des*Arg⁹-BKproduite dans l'échantillon de plasma du patient,
d) on détermine la quantité totale de *des*Arg⁹-BK produite chez le patient, et
e) on détermine la quantité nécessaire dudit antagoniste, à partir de la détermination réalisée à l'étape d), cette quantité étant équimolaire avec la quantité de *des*Arg⁹-BK produite.

Selon cet aspect de l'invention, le dosage de l'étape b) est un dosage immunoenzymatique compétitif.

Selon un autre aspect de l'invention, on utilise, pour le dosage de l'étape b), des anticorps anti- *des*Arg⁹-BK, des traceurs DIG couplés à la
desArg⁹-BK, des anticorps anti-DIG couplés à une enzyme et des substrats de l'enzyme.

Selon unautre aspect de l'invention encore, l'étape b) comprend elle-même les étapes suivantes :
- on immobilise des anticorps anti-*des*Arg⁹-BKdilués dans du tampon de fixation sur un support approprié comportant plusieurs cavités ;
- à intervalle de temps régulier, on prélève une partie aliquote de l'échantillon activé et on incube une cavité du support avec ladite partie aliquote de l'échantillon et une quantité déterminée d'un traceur DIG couplé à la *des*Arg⁹-BK pendant une période de temps déterminée;
- après lavage, on incube le support avec des anticorps anti-DIG couplés à une enzyme ;
- après lavage, on incube le support avec une quantité déterminée du substrat de l'enzyme ;
- on mesure l'activité enzymatique par mesure colorimétrique en cinétique.
L'enzyme couplée aux anticorps anti-DIG peut par exemple être la phosphatase alkaline ou la peroxidase HRP.

Selon un aspect de l'invention, l'antagoniste de la desArg⁹-BK est le R954, c'est-à-dire le peptide Ac-Orn-[Oic², αMePhe⁵, D-βNal⁷, Ile⁸] *des*Arg⁹-BK.

### Méthode de diagnostic d'un angioedème à forte kininoformation

La présente invention concerne également une méthode de diagnostic d'un angioedème à gain de fonction chez un patient. Cette méthode comprend les étapes suivantes :
a) on active la libération de BK par mise en contact d'un échantillon du plasma du patient avec une quantité déterminée d'un agent électronégatif,
b) on dose la quantité de BK libérée selon l'étape a), qui correspond à la réserve de BK dans l'échantillon de plasma du patient, et
c) on compare la valeur obtenue à l'étape b) avec une valeur contrôle qui correspond à la réserve de BK disponible dans un échantillon de plasma d'un témoin sain de même catégorie de manière à déterminer la quantité de BK produite dans l'échantillon de plasma du patient, et
d) éventuellement, on détermine la quantité totale de BK produite chez le patient.
e) une forte diminution de la quantité de BK libérée par activation dans l'échantillon de plasma du patient comparativement à la quantité déterminée dans les mêmes conditions dans l'échantillon de plasma du témoin atteste d'un angioedème avec forte kininoformation, dont l'angiodème à gain de fonction.

Par « patient », on entend tout sujet pour lequel l'approximation qualitative pour la capacitéà produire des kinines à partir du kininogène HK est nécessaire au diagnostic de susceptibilité.

### Kit

La présente description mentionne également un kit ou une trousse pour la mise en oeuvre de l'une des méthodes de détermination selon la présente invention, comprenant :
- un premier réactif consistant en des anticorps anti-BK ou anti-*des*Arg⁹-BK,
- un second réactif consistant en un traceur DIG couplé à la BK ou couplé à la *des*Arg⁹-BK,
- un troisième réactif consistant en des anticorps anti-DIG couplés à une enzyme,
- un quatrième réactif consistant en un substrat de l'enzyme.

### Exemples

### Exemple 1 - activité kininogénase spontanée et activabilité des proenzymes

### Matériels et méthodes

### a) Echantillonnage

Plasma citrate. 2 × 4.5 mL de sang sont prélevés par ponction veineuse et recueillis dans des tubes BD Vacutainer® bouchon bleu-clair contenant 0,1 mol/L Na citrate (type 366415). Le plasma est recueilli après centrifugation (20°C, 15 min, 2500×g).

### b) Activité Kininogénase du plasma :

Principe : L'activité des enzymes coupant kininogèneHK (kininogénases) est estimée vis-à-vis du peptide Pro-Phe-Arg-para-nitroanilide, représentatif du C-terminus de la BK (résidus 285-289 de HK ; séquence peptidique identifiée P01042). Les proenzymes correspondant sont activables par des agents
électronégatifs (sulfate de dextran par exemple). Elle est évaluée par méthode amidolytique à l'aide du substrat H-D-Pro-Phe-Arg- pNA dont le clivage est détectable par changement de couleur (libération du groupement chromophore-para-nitroanilinep-NA) permettant une mesure photométrique à 405 nm (ε 405 nm = 8 800 M⁻¹•cm⁻¹).
Deux activités sont mesurées sur les échantillons plasmatiques :

### 1- Activité spontanée du plasma

### 2- Degré d'activabilité des proenzymes par le sulfate de dextran

**Tableau 1. Valeurs de références des activités enzymatiques de la kininoformation.**

| | Activité kininogénases spontanée plasma (nmol/ml/min) | Potentiel activabilité D.S. (nmol/ml/min) | Rapport (%) |
|---|---|---|---|
| Sujet masculin (18-58 ans) | (2.3-5.6) médiane 3.6 | (2225-4273) 3288 | (0.07-0.17) 0.11 |
| Sujet féminin (18-57 ans) | (2.4-10.7) médiane 4.3 | (2422-4560) 3523 | (0.07-0.29) 0.13 |

### Exemple 2 - capacité de formation des kinines chez les patients souffrant d'angioedème

### Dosage des kinines BK et desArg⁹-BK :

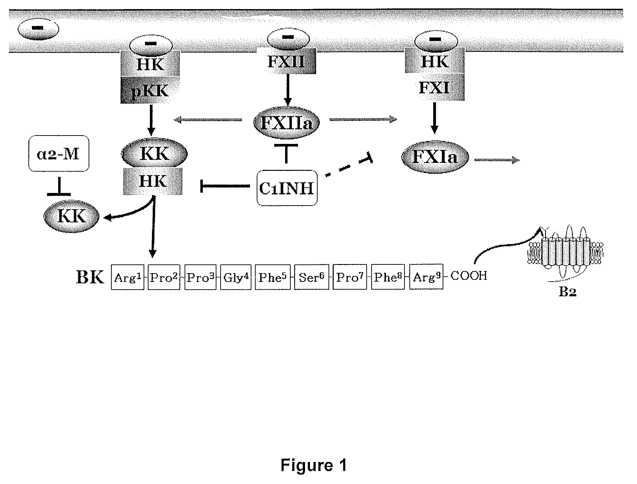

La capacité de la kininoformationpar le plasma des patients est examinée dans le contexte des échantillons plasmatiques des patients souffrant d'angioedème par la cinétique de la production plasmatique de BK (figure 1A). Cette mesure peut être complétée par celle de desArg⁹-BK qui rend compte du catabolisme de la BK (figure 1B).

### Matériel et méthodes

Le volume de plasma (500 µl) est activé *in vitro* par le sulfate de dextran (SIGMA, 25 µg/ml concentration finale), un activateur riche en charges négatives. Régulièrement (0 min, 1 min, 2 min, 4 min, 6 min, 8 min, 16 min, 32 min, 60 min), 50 µl sont prélevés, précipités par 10 volumes d'éthanol et centrifugés (4°C, 30 min, 3500xg). Après évaporation des surnageants (Speed Vac) et reprise du contenu des tubes par 50 µl d'eau distillée, les concentrations de BK (A) et *des*Arg⁹-BK(B) sont mesurées par le test ELISA par compétition avec le traceur peptidique marqué par DIG (ROCHE) et à l'aide des anticorps spécifiques. L'absorbance est mesurée au spectrophotomètre, les essais sont repérés par rapport à une gamme étalon.

Ces données qualifient l'angioedème, avec l'accès à des paramètres stratégiques sur les ligands des récepteurs de la perméabilité vasculaire :
- la quantité (mol) de kinines(BK et *des*Arg⁹-BK)produites à partir du kininogène HK, par dosage de la réserve de BK et comparaison avec une valeur contrôle, plus précisément par un calcul de l'aire sous la courbe AUC (évaluation de l'importance de la coupure du kininogène HK),
- la quantité de médicament antagoniste du récepteur B2 (icatibant) nécessaire pour déplacer la BK produite,
- le temps de demi-formation de la BK par le plasma du patient (évaluation de la kininoformation),
- la demi-vie de chacune des kinines dans le milieu plasmatique (évaluation du catabolisme).

### Exemple 3 - Patient en situation de crise

### 1. Situation clinique

Un sujet féminin, âgé de 24 ans, est admis pour angioedème en service d'urgence. Il présente des crampes abdominales, une gêne respiratoire, un AO des paupières.

Le sujet est non porteur de la mutation faux-sens 983A/G de l'exon 9 du gène *F12*, sans médicament, fonction normale de C1Inh.

### 2. Biologie

### 2.1. Forte kininoformation avec activité spontanée élevée, catabolisme des kinines normal (tableau 2)

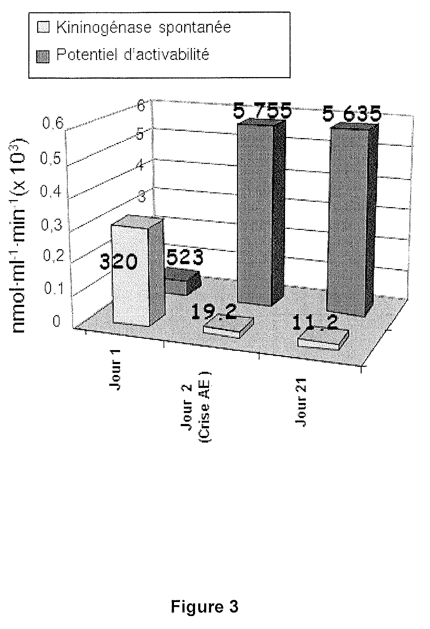

### 2.2. Cinétique des événements biologiques avant crise, en crise, après restauration (figure 3)

Observation : Les proenzymes sont rapidement reconstitués par la synthèse hépatique après activation avec le temps de demi-reconstitution de moins de 6 heures.

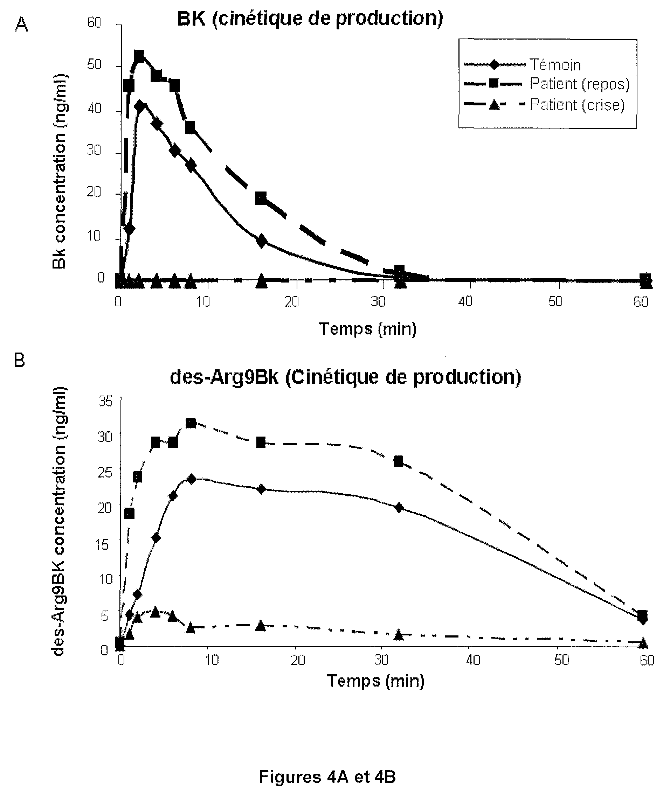

### 2.3. Evolution de la capacité du kininogène HK à produire BK et desArg⁹-BK dans les deux situations avant et après la crise d'angioedème à gain de fonction (figures 4A, 4B)

Observation : Le kininogène HK subit une coupure extensive par les protéases endothéliales. Cette coupure au cours de la crise d'angioedème se traduit par la perte de production de BK et *des*Arg⁹-BK.Cela signifie la perte fonctionnelle du kininogène HK. La fonction et l'intégrité du kininogèneHK sont restaurées lentement avec le temps de demi-reconstitution de plus de 48 heures.

### 3. Résultats

### 3.1. Kininoformation spontanée : activité spontanée élevée

**Tableau 2. Activités enzymatiques de la kininoformation au cours des événements cliniques d'angioedème.**

| | Activité kininogénase spontanée plasma (nmol/ml/min) | Potentiel activabilité D.S. (nmol/ml/min) | Rapport (%) |
|---|---|---|---|
| Echantillon n° 1 - Prélèvement au cours d'une consultation | 320 | 523 | 36.98 |
| Echantillon n° 2 - Prélèvement en crise 33h après le 1 er prélèvement | 19.2 | 5755 | 0.33 |
| Echantillon n° 3 - Prélèvement de contrôle après 21 jours | 11.2 | 5635 | 0.20 |

Les activités des enzymes du catabolisme des kinines BK et *des*Arg⁹-BK sont normales (données non présentées).

Pour évaluer la possible contribution des mastocytes, le niveau de la tryptase a été exploré sur les échantillons plasmatiques 1 et 2:
3.65 µg/l (référence <13.5 µg/l)
4.30 µg/l (référence <13.5 µg/l)
et sur un échantillon 3 indépendant de l'épisode : 3.1 µg/l (référence <13.5 µg/l)
Il n'y a pas de modification du niveau de la tryptase au cours du temps, ce qui exclut toute participation mastocytaire.

### 2. Cinétique des événements biologiques avant la crise, pendant la crise et à distance de 21 jours de la crise

Il s'agit de la première observation du développement des enzymes et des kinines au cours de l'épisode d'angioedème et à distance de cet épisode, chez un sujet non déficitaire pour C1Inh et non porteur de la mutation faux-sens c.983A/G (p.Thr328Lys) sur le gène *F12*.
Originalité : première observation sur la kininoformation dans les heures précédant la symptomatologie et pendant la symptomatologie.

### 3. Interprétation des données expérimentales

- La kininoformation est un préalable à la crise, environ 24-36 heures avant l'oedème. L'activité kininogénase spontanée avec la production de BK représente un élément diagnostic précoce, anticipant l'événement clinique.
- La rapide reconstitution des proenzymes est cohérente avec la forte production hépatique, mobilisant rapidement la prékallicréine et le proenzyme facteur Hageman au niveau endothélial.
- La baisse de l'activabilitédes proenzymes apporte la preuve solide *ex vivo* de la rapide activation endothéliale des proenzymes de la phase contact.
- Le niveau normal de la tryptase dans les échantillons écarte la composante mastocytaireet les protéases associées dans ce phénomène.

Explication du phénomène biologique :
- La forte activation des proenzymes se traduit par la protéolyse vis-à-vis du kininogène HK.
- L'activité protéolytique vis-à-vis du kininogène HK est transitoire, sa dégradation par coupure protéolytique est très importante.
- Phénomène clinico-biologique : En conséquence la diffusion de la bradykinine et de la *des*Arg9-BK entraîne l'activation des récepteurs B2 et B1 et déclenche la perméabilité vasculaire.

### Interprétation médicale

- La symptomatologie d'angioedème est postérieure à l'activité enzymatique capable de produire les kinines ; le délai de 24-36 heures est le temps nécessaire à la diffusion de l'eau vers les tissus.
- La présente observation documente de façon pédagogique l'angioedème clinique dans la situation à gain de fonction des kininogénases.

### Conclusion

- Ces tests biologiques sont applicables à l'examen d'une situation d'angioedème à gain de fonction.
- Ils doivent être choisis pour apporter l'argumentaire nécessaire à établir le diagnostic étiopathogénique, décisionnel pour l'application d'une thérapeutique ciblée.

### Exemple 4 - Patients en situation de crise-diagnostic et détermination de la quantité d'antagoniste de la BK nécessaire pour stopper les effets de l'angioedème

### a) sujet féminin de 24 ans non porteur de la mutation faux-sens 983A/G de l'exon 9 du gène F12, sans médicament, fonction normale de C1Inh.

Dans un premier temps, on procède audosage de la quantité de BK libérée (ou produite) dans un échantillon de plasma citrate du patient par activation *in vitro* de la phase contact. La valeur obtenue correspond à la réserve de BK, ou quantité de BK disponible, dans l'échantillon de sang total du patient (volume : 500µL). Ce dosage est réalisé tel que décrit dans l'exemple 2.

La Figure 4A montre que la production résiduelle de la BKBK est très faible chez le patient en situation pathologique (échantillon 1, patient crise), comparativement au témoin.

La Figure 4A montre également que hors de la situation pathologique (échantillon 3, patient repos), le patient dispose de la capacité de production de la BK comparable au témoin.

Dans un second temps, on compare la valeur obtenue à l'étape a) avec une valeur contrôle correspondant au dosage de la réserve de BK dans un échantillon de sang total d'un témoin sain de même catégorie (figure 4A témoin). Cette valeur contrôle correspond à l'aire sous la courbe AUC de BK (figure 4A).

Les courbes 4A du patient et du témoin sont lissées par la méthode des B-splines. Les aires sous les courbes (AUC) sont représentatives de la quantité de BK libérée par activation de la phase contact ; elles sont calculées avec ou sans lissage par la méthode des trapèzes.

| | **Après lissage** | **Sans lissage** |
|---|---|---|
| AUC 3A3 (patient, situation au repos, échantillon 3) | 512.0 ng/ml | 751.75 ng/ml |
| AUC 3A1 (patient, situation pathologique, échantillon 1) | 2.74 ng/ml | 3.9 ng/ml |
| Témoin | 564.68 ng/ml | 465.18 ng/ml |

Selon une troisième étape, le calcul des différences entre la situation au repos (échantillon 3) et la situation pathologique (échantillon 1) donne lieu à la connaissance de la production de BK (ng/ml) produite par activation de l'échantillon plasmatique. Ceci permet de calculer la quantité de BK (poids moléculaire 1 060) produite dans le volume échantillon du patient pendant le développement pathologique.

| | **Après lissage** | **Sans lissage** |
|---|---|---|
| AUC 3A3 - AUC 3A1 (patient) | 509.2 ng/ml | 747.8 ng/ml |
| | 480.4 nmol/l | 705 nmol/l |

Rapportée au volume plasmatique total, la différence AUC 3A3 - AUC 3A1 correspond donc à la quantité de BK produite pendant la phase active de la symptomatologie :

**Poids du sujet 54 kg, Volume sanguin 4.1 I, Volume plasmatique 2.25 I**

| | **Après lissage** | **Sans lissage** |
|---|---|---|
| AUC 3A3 - AUC 3A1 (patient) | 1.15 mg | 1.68 mg |
| | 1 081 nmol | 1 586.2 nmol |

A ce stade, les données sont suffisantes pour dire que la patiente présente un angioedeme à gain de fonction.

Enfin, on procède à la détermination de la quantité nécessaire dudit antagoniste. La quantité de médicament à appliquer doit être au minimum de 1 081 nmol, soit environ 1,4 mg d'antagoniste icatibant (poids moléculaire 1 305). L'icatibant est conditionné sous forme d'une solution injectable de 3ml à 10 mg/ml. Ceci implique qu'aujourd'hui, les patients arrivant aux services des urgences en situation de crise recoivent une quantité d'antagoniste de 30mg. La méthode de la présente invention indique qu'une quantité nettement inférieure est suffisante pour stopper les effets de l'angioedème. Ceci a en outre comme avantage de dimineur les effets indésirables associés à l'administration d'une quantité d'icatibant trop importante.
b) sujet féminin de 61 ans, non porteur de mutation sur les gènes *SERPING1* et *F12* développant une pathologie d'angioedème sous déclencheur agoniste oestrogénique (raloxifène) ; forte activité kininogénase spontanée au moment de la crise.
c) sujet féminin de 33 ans, non porteur de mutation sur les gènes *SERPING1* et *F12*, développant une pathologie d'angioedème sous déclencheur oestrogène (ethinyl-oestradiol) ; forte activité kininogénase spontanée au moment de la crise.

Les échantillons plasmatiques des sujets b) et c) ont été recueillis pendant et hors la période active de la maladie.
Ils ont été soumis à la méthode de détermination de la quantité d'antagoniste BK nécessaire pour stopper la crise d'angioedème selon la présente invention :
- activation par le sulfate de dextran (25 µg/ml) en tant qu'agent électronégatif ;
- dosage des quantités de BK libérée au cours du temps pendant les 60 minutes qui suivent l'activation (échantillon prélevé pendant la crise et échantillon prélevé en dehors de la crise qui correspond à une valeur contrôle) et comparaison des valeurs obtenues ;

- détermination de la quantité totale de BK produite ;
- détermination de la quantité d'antagoniste nécessaire pour stopper la crise d'angioedème.

| | Sujet b) | Sujet c) |
|---|---|---|
| Quantité de BK produite dans l'échantillon de plasma du patient | 448.5 ng/ml | 437 |
| Quantité totale de BK produite | 1.17 mg | 1.08 mg |
| chez le patient | 1.10 nmol | 1.01 nmol |
| Quantité d'antagoniste nécessaire pour déplacer la BK produite, stopper la crise d'angioedeme | 1,43 mg | 1,32 mg |

La quantité de médicament à appliquer doit être au minimum de 1,43 mg d'antagoniste icatibant (poids moléculaire 1 305) pour le patient b) et de 1,32 mg pour le patient c).

### Exemple 5 - Patient en situation de crise - détermination de la quantité d'antagoniste de desArg⁹-BKnécessaire pour stopper les effets de l'angioedème

On procède audosage de la quantité de *des*Arg⁹-BK produite dans un échantillon de plasma citrate du patient par activation *in vitro* de la phase contact.

La Figure 4B montre que la production résiduelle de la desArg⁹-BK est très faible chez le patient en situation pathologique (échantillon 1), comparativement au témoin.

La Figure 4B montre que hors de la situation pathologique (échantillon 3), le patient dispose de la capacité de production de la *des*Arg⁹-BK comparable au témoin.

Les aires sous les courbes (AUC) de la figure 4B sont représentatives de la quantité de *des*Arg⁹-BK libérée par activation de la phase contact ; elles sont calculées par la méthode des trapèzes sans lissage.

| | **Sans lissage** |
|---|---|
| AUC 3B3 (patient, situation au repos, échantillon 3) | 1 299.2 ng/ml |
| AUC 3B1 (patient, situation pathologique, échantillon 1) | 125.6 ng/ml |
| Témoin | 957.9 ng/ml |

Le calcul des différences entre la situation au repos (échantillon 3) et la situation pathologique (échantillon 1) donne lieu à la connaissance de la production de *des*Arg⁹-BK (ng/ml) produite par activation de l'échantillon plasmatique. Ceci permet de calculer la quantité de *des*Arg⁹-BK (poids moléculaire 904) produite dans le volume échantillon du patient pendant le développement pathologique.

| | **Sans lissage** |
|---|---|
| AUC 3B3 - AUC 3B1 (patient) | 1 173.6 ng/ml |
| | 1 298 nmol/l |

Rapportée au volume plasmatique total, la différence AUC 3B3 - AUC 3B1 correspond donc à la quantité de *des*Arg⁹-BK produite pendant la phase active de la symptomatologie :

| | **Sans lissage** |
|---|---|
| AUC 3B3 - AUC 3B1 (patient) | 2.64 mg |
| | 2 920 nmol |

La quantité de *des*Arg⁹-BK produite est deux fois plus élevée que celle de la BK, ce qui correspond à l'accumulation de *des*Arg⁹-BK, affectée par une demi-vie prolongée.

## Revendications

1. Méthode de détermination de la quantité d'antagoniste de la BK nécessaire pour stopper les effets d'un angioedème à partir d'un échantillon de plasma d'un patient comprenant les étapes suivantes :
a) on active la libération de BK par mise en contact de l'échantillon du plasma du patient avec une quantité déterminée d'un agent électronégatif,
b) on dose la quantité de BK libérée selon l'étape a), qui correspond à la réserve de BK dans l'échantillon de plasma du patient,
c) on compare la valeur obtenue à l'étape b) avec une valeur contrôle qui correspond à la réserve de BK disponible dans un échantillon de plasma d'un témoin sain de même catégorie, de manière à déterminer la quantité de BK produite dans l'échantillon de plasma du patient,
d) on détermine la quantité totale de BK produite chez le patient, et
e) on détermine, à partir de la valeur obtenue à l'étape d), la quantité nécessaire dudit antagoniste, cette quantité étant équimolaire avec la quantité de BK produite.

2. Méthode selon la revendication 1, dans laquelle le dosage de l'étape b) est un dosage immunoenzymatique compétitif.

3. Méthode selon la revendication 1 ou 2, dans laquelle on utilise, pour le dosage de l'étape b) des anticorps anti-BK, des traceurs DIG couplés à la BK, des anticorps anti-DIG couplés à une enzyme et des substrats de l'enzyme.

4. Méthode selon la revendication1 ou 2, dans laquelle l'étape a) comprend elle-même les étapes suivantes :
- on immobilise des anticorps anti-BK dilués dans du tampon de fixation sur un support approprié comportant plusieurs cavités ;
- à intervalle de temps régulier, on prélève une partie aliquote de l'échantillon activé et on incube une cavité du support avec ladite partie aliquote de l'échantillon et une quantité déterminée d'un traceur DIG couplé à la BK pendant une période de temps déterminée;
- après lavage, on incube le support avec des anticorps anti-DIG couplés à une enzyme ;
- après lavage, on incube le support avec une quantité déterminée du substrat de l'enzyme ;
- on mesure l'activité enzymatique par mesure colorimétrique directe.

5. Méthode selon la revedication 4, dans laquelle l'enzyme couplée aux anticorps anti-DIG est la phosphatase alkaline ou la peroxidase.

6. Méthode selon l'une quelconque des revendications précédentes, dans laquelle l'antagoniste de la BK est l'icatibant HOE 140.

7. Méthode selon l'une quelconque des revendications précédentes, dans laquelle l'agent électonégatif est choisi parmi le sulfate de dextran, les billes de verre et le silicate d'aluminium.

8. Méthode de diagnostic d'un angioedème avec forte kininoformation chez un patient comprenant :
a) on active la libération de BK par mise en contact d'un échantillon du plasma du patient avec une quantité déterminée d'un agent électronégatif,
b) on dose la quantité de BK libérée selon l'étape a), qui correspond à la réserve de BK dans l'échantillon de plasma du patient, et
c) on compare la valeur obtenue à l'étape b) avec une valeur contrôle qui correspond à la réserve de BK disponible dans un échantillon de plasma d'un témoin sain de même catégorie, de manière à déterminer la quantité de BK produite dans l'échantillon de plasma du patient,
d) éventuellement, on détermine la quantité totale de BK produite chez le patient,
e) une forte diminution de la quantité de BK libérée par activation dans l'échantillon de plasma du patient comparativement à la quantité déterminée dans les mêmes conditions dans l'échantillon de plasma du témoin atteste d'un angioedème avec forte kininoformation, dont l'angiodème à gain de fonction.

## Patentansprüche

1. Verfahren zur Bestimmung der Menge an BK-Antagonist, die notwendig ist, um die Auswirkungen eines Angioödems zu stoppen, ausgehend von einer Plasmaprobe eines Patienten, welches folgende Schritte umfasst:
a) Aktivieren der BK-Freisetzung durch Inkontaktbringen der Plasmaprobe des Patienten mit einer bestimmten Menge einer elektronegativen Substanz,
b) Dosieren der gemäß Schritt a) freigesetzten BK-Menge, welche der BK-Reserve in der Plasmaprobe des Patienten entspricht,
c) Vergleichen des in Schritt b) erhaltenen Werts mit einem Kontrollwert, welcher der in einer Plasmaprobe einer gesunden Vergleichsperson der gleichen Kategorie verfügbaren BK-Reserve entspricht, um die in der Plasmaprobe des Patienten produzierte BK-Menge zu bestimmen,
d) Bestimmen der beim Patienten produzierten BK-Gesamtmenge, und
e) Bestimmen, ausgehend von dem in Schritt d) erhaltenen Wert, der notwendigen Menge des besagten Antagonisten, wobei diese Menge mit der produzierten BK-Menge äquimolar ist.

2. Verfahren nach Anspruch 1, wobei die Dosierung in Schritt b) eine immunenzymatisch kompetitive Dosierung ist.

3. Verfahren nach Anspruch 1 oder 2, wobei für die Dosierung in Schritt b) Anti-BK-Antikörper, an das BK gekoppelte DIG-Marker, an ein Enzym gekoppelte Anti-DIG-Antikörper und Substrate des Enzyms verwendet werden.

4. Verfahren nach Anspruch 1 oder 2, wobei Schritt a) seinerseits die folgenden Schritte umfasst:
- Immobilisieren der in Fixierpuffer verdünnten Anti-BK-Antikörper auf einem geeigneten Träger, der mehrere Kavitäten umfasst;
- in regelmäßigem zeitlichen Abstand Entnahme eines aliquoten Teils der aktivierten Probe und Inkubieren einer Kavität des Trägers mit besagtem aliquoten Teil der Probe und einer bestimmten Menge eines an das BK gekoppelten DIG-Markers während eines bestimmten Zeitraums;
- nach Waschen Inkubieren des Trägers mit an ein Enzym gekoppelten Anti-DIG-Antikörpern;
- nach Waschen Inkubieren des Trägers mit einer bestimmten Menge des Substrats des Enzyms;
- Messen der enzymatischen Aktivität mittels direkter kolorimetrischer Messung.

5. Verfahren nach Anspruch 4, wobei das an die Anti-DIG-Antikörper gekoppelte Enzym alkalische Phosphatase oder Peroxidase ist.

6. Verfahren nach einem der vorstehenden Ansprüche, wobei der BK-Antagonist Icatibant HOE 140 ist.

7. Verfahren nach einem der vorstehenden Ansprüche, wobei die elektronegative Substanz ausgewählt ist aus Dextransulfat, Glasperlen und Aluminiumsilikat.

8. Verfahren zur Diagnose eines Angioödems mit gesteigerter Kinin-Bildung bei einem Patienten, welches umfasst:
a) Aktivieren der BK-Freisetzung durch Inkontaktbringen einer Plasmaprobe des Patienten mit einer bestimmten Menge einer elektronegativen Substanz,
b) Dosieren der gemäß Schritt a) freigesetzten BK-Menge, welche der BK-Reserve in der Plasmaprobe des Patienten entspricht, und
c) Vergleichen des in Schritt b) erhaltenen Werts mit einem Kontrollwert, welcher der in einer Plasmaprobe einer gesunden Vergleichsperson der gleichen Kategorie verfügbaren BK-Reserve entspricht, um die in der Plasmaprobe des Patienten produzierte BK-Menge zu bestimmen,
d) gegebenenfalls Bestimmen der beim Patienten produzierten BK-Gesamtmenge,
e) eine starke Verringerung der durch Aktivierung in der Plasmaprobe des Patienten freigesetzten BK-Menge im Vergleich zu der unter den gleichen Bedingungen in der Plasmaprobe der Vergleichsperson bestimmten Menge bestätigt ein Angioödem mit gesteigerter Kinin-Bildung, darunter das Angioödem mit Funktionszugewinn.

## Claims

1. A method for determining the amount of BK antagonist required to stop the effects of angioedema from a plasma sample of a patient comprising the following steps:
a) activating the release of BK by contacting the sample of the plasma of the patient with a determined amount of an electronegative agent,
b) dosing the amount of released BK according to step a) which corresponds to the BK reserve in the plasma sample of the patient,
c) comparing the value obtained in step b) with a control value which corresponds to the BK reserve available in a plasma sample from a healthy control of the same category, so as to determine the BK amount produced in the plasma sample of the patient,
d) determining the BK total amount produced in the patient, and
e) determining, from the value obtained in step d), the required amount of said antagonist, this amount being equimolar with the produced BK amount.

2. The method according to claim 1, wherein the dosing of step b) is a competitive immunoenzymatic dosing.

3. The method according to claim 1 or 2, wherein for the dosing of step b), anti-BK antibodies, DIG markers coupled to BK, anti-DIG antibody coupled to an enzyme and substrates of the enzyme are used.

4. The method according to claim or 2, wherein step a) itself comprises the following steps:
- immobilizing anti-BK antibodies diluted in bonding plug on a suitable support including a plurality of cavities;
- at regular time interval, an aliquot portion of the activated sample is collected and a cavity of the support is incubated with said aliquot portion of the sample and a determined amount of a DIG marker coupled to BK during a determined period of time;
- after washing, the support is incubated with anti-DIG antibodies coupled to an enzyme;
- after washing, the support is incubated with a determined amount of the substrate of the enzyme;
- measuring the enzyme activity by direct colorimetric measurement.

5. The method according to claim 4, wherein the enzyme coupled to the anti-DIG antibodies is alkaline phosphatase or peroxidase.

6. The method according to any one of the preceding claims, wherein the BK antagonist is icatibant HOE 140.

7. A method according to any one of the preceding claims, wherein the electronegative agent is selected from dextran sulfate, glass beads, and aluminum silicate.

8. A diagnostic method of an angioedema with strong kininoformation in a patient comprising:
a) activating the BK release by contacting a sample of the plasma of the patient with a determined amount of an electronegative agent,
b) dosing the released BK amount according to step a) one dose), which corresponds to the BK reserve in the plasma sample of the patient, and
c) comparing the value obtained in step b) with a control value which corresponds to the BK reserve available in a plasma sample from a healthy control of the same category, so as to determine the BK amount produced in the plasma sample of the patient,
d) optionally, determining the BK total amount produced in the patient,
e) strongly decreasing the BK amount released by activation in the the plasma sample of the patient compared to the determined amount under the same conditions in the plasma sample of the witness attests an angioedema with strong kininoformation, including gain-of-function angioedema.
